Europäisches Patentamt

European Patent Office

Office européen des brevets

(19)

(11) Veröffentlichungsnummer: **0 252 242**
**B1**

(12) **EUROPÄISCHE PATENTSCHRIFT**

(45) Veröffentlichungstag der Patentschrift:
28.11.90

(51) Int. Cl.⁵: **C07D 207/267, B01J 23/74,**
**B01J 23/78, B01J 23/86**

(21) Anmeldenummer: **87106782.3**

(22) Anmeldetag: **11.05.87**

(54) Verfahren zur Herstellung von C1- bis C6-N-Alkylpyrrolidonen aus Bernsteinsäureanhydrid bzw. C1- bis C6-N-Alkylsuccinimid.

(30) Priorität: 07.07.86 DE 3622759

(43) Veröffentlichungstag der Anmeldung:
13.01.88 Patentblatt 88/2

(45) Bekanntmachung des Hinweises auf die Patenterteilung:
28.11.90 Patentblatt 90/48

(84) Benannte Vertragsstaaten:
AT BE CH DE FR GB IT LI NL

(56) Entgegenhaltungen:
EP-A- 0 027 022
DE-A- 1 620 191
DE-C- 830 194
GB-A- 1 439 558

BEILSTEINS HANDBUCH DER ORGANISCHEN
CHEMIE, 4. Auflage, E III/IV, Band 21, 4.
Teil, 1978 SPRINGER-VERLAG, Berlin, Heidelberg, New
York, Seite 3145

Die Akte enthält technische Angaben, die nach dem
Eingang der Anmeldung eingereicht wurden und die
nicht in dieser Patentschrift enthalten sind.

(73) Patentinhaber: HÜLS AKTIENGESELLSCHAFT, - RSP
Patente / PB 15 - Postfach 13 20, D-4370 Marl 1(DE)

(72) Erfinder: zur Hausen, Manfred, Dr., Hoechser Strasse 1,
D-4370 Marl(DE)
Erfinder: Otte, Werner, Dr., Eichenstück 55,
D-4270 Dorsten 11(DE)

ACTORUM AG

**Beschreibung**

Die Erfindung betrifft ein Verfahren zur Herstellung von $C_1$- bis $C_6$-N-Alkylpyrrolidonen aus Bernsteinsäureanhydrid bzw. $C_1$- bis $C_6$-N-Alkylsuccinimid.

N-Alkylpyrrolidone sind als Lösemittel oder Zwischenprodukte von Bedeutung.

Nach bekannten Verfahren erhält man N-Alkylpyrrolidone aus den entsprechenden N-Alkylsuccinimiden durch elektrochemische Reduktion (Beilstein E III/IV 21, Seite 3145), aus Pyrrolidin-2-on und Methanol an $Al_2O_3$ (DE-PS 830 194), aus Dihydrofuran-2-on und Methylamin (Beilstein E III/IV 21, Seite 3145), aus Bernsteinsäure über N-Methylsuccinimid in Gegenwart von Ra-Ni und Dioxan bei 200 bis 215°C und 200 bis 220 bar (Khim. Prom, 1963 (7), Seiten 491 bis 492) oder aus Succinimid in Gegenwart des Alkohols, der der gewünschten Alkylgruppe entspricht, an Kobalt- oder Nickel-Katalysatoren bei 200 bis 280°C und 200 bis 300 bar (SU-PS 259 889) sowie nach der DE-AS 1 620 191, bei der Bernsteinsäure mit Alkylamin und Wasserstoff in Gegenwart von Raneymetallen, Co-, Ni-, Ru- oder Pd-Katalysatoren bei 200 bis 300°C und Drucken von 50 bar umgesetzt wird. Entgegen den Angaben in der Beschreibung (Spalte 3, Zeile 3) kann nach den Beispielen bei der Durchführung dieses Verfahrens offensichtlich nicht auf den Einsatz eines Löse- oder Verdünnungsmittels (Wasser oder Dioxan) verzichtet werden.

Bei den genannten Verfahren werden bestenfalls Ausbeuten an Alkylpyrrolidon von 80% erhalten.

Ausgehend von N-Methylsuccinimid (Khim. Prom. 491-2, 1963) wird an Raney-Ni sogar nur eine Ausbeute von 71,6 % erreicht.

Es bestand daher die Aufgabe, ein Verfahren zu finden, das aus den leicht verfügbaren Rohstoffen Bernsteinsäureanhydrid sowie $C_1$- bis $C_6$-N-Alkylaminen oder $C_1$- bis $C_6$-N-Alkylsuccinimid in wirtschaftlicher Weise und guten Ausbeuten zu den entsprechenden $C_1$- bis $C_6$-N-Alkylpyrrolidonen führt.

Diese Aufgabe wird erfindungsgemäß entsprechend den Angaben der Patentansprüche gelöst.

Überraschenderweise wurde gefunden, daß man bei dem erfindungsgemäßen Verfahren $C_1$- bis $C_6$-N-Alkylpyrrolidone in Ausbeuten von über 85 % erhält, wenn man Ni-Katalysatoren einsetzt, die als Aktivatoren und/oder Stabilisatoren geringe Mengen von 0,1 bis 3 % an Erdalkalioxiden (z. B. von Mg, Ca) und/oder Eisenoxid und/oder Chromoxid enthalten. Man kann ein oder mehrere Erdalkalioxide einsetzen. Als Eisenoxid setzt man vorzugsweise $Fe_2O_3$ ein, als Chromoxid vorzugsweise $Cr_2O_3$ oder $Cr_3O_4$. Insbesondere setzt man die Aktivatoren in Mengen von 1,5 bis 2 % ein.

Besonders vorteilhafte Ergebnisse werden erhalten, wenn man die Erdalkalioxide gemeinsam mit Eisenoxid und Chromoxid, vorzugsweise mit $Fe_2O_3$ und $Cr_2O_3$ bzw. $Cr_3O_4$, insbesondere in Mengen von 1,5 bis 2 % einsetzt. Die erfindungsgemäßen Katalysatoren werden nach dem Stand der Technik hergestellt.

Beim erfindungsgemäßen Verfahren arbeitet man bei Temperaturen von 180 bis 250 °C, vorzugsweise im Temperaturbereich von 200 bis 230 °C.

Der Druck sollte beim erfindungsgemäßen Verfahren oberhalb von 100 bar liegen. Zweckmäßigerweise wendet man Drucke oberhalb von 200 bar an, wobei man bevorzugt zwischen 250 und 320 bar arbeitet.

Das erfindungsgemäße Verfahren kann sowohl diskontinuierlich als auch kontinuierlich ausgeführt werden, z. B. in einem Rührautoklaven oder einem Reaktionsrohr. Die zur Hydrierung erforderlichen Apparaturen sind Stand der Technik. Es besteht die Möglichkeit, das erfindungsgemäße Verfahren in der Sumpfphase oder Rieselphase zu betreiben.

Im diskontinuierlichen Betrieb wird üblicherweise in der Sumpfphase in einem Autoklaven in Gegenwart eines pulverförmigen Katalysators gearbeitet.

Besonders vorteilhaft ist die kontinuierliche Arbeitsweise bei dem erfindungsgemäßen Verfahren. Üblicherweise führt man die Ausgangsstoffe flüssig über den im Reaktionsrohr befindlichen stückig angeordneten Katalysator, z. B. nach dem Rieselphasenprinzip, während der Wasserstoff im Gleich- oder Gegenstrom durch das Reaktionsrohr geleitet wird. Vorteilhaft ist es, den überschüssigen Wasserstoff im Kreis zu führen.

Die nachfolgenden Beispiele sollen das erfindungsgemäße Verfahren veranschaulichen.

Beispiel 1

Ein 400 ml-Durchflußreaktor wird mit einem Ni-Katalysator befüllt (NiO: 80 %; CaO: 0,5 %, MgO: 0,6 %; $Fe_2O_3$: 0,2 %; $Cr_3O_4$: 0,5 %; $SiO_2$: 9 %; $Al_2O_3$: 7 %) und stündlich mit 80 ml N-Methylsuccinimid beschickt. Die Hydrierung erfolgt bei 300 bar Gesamtdruck und 200 °C. Die Ergbnisse sind in der Tabelle aufgeführt.

Beispiel 2

Ausführung wie in Beispiel 1, jedoch wird die Temperatur auf 250 °C erhöht. Die Ergebnisse sind in der Tabelle aufgeführt.

Vergleichbare Ergebnisse werden erhalten, wenn man anstelle von N-Methylsuccinimid Bernsteinsäureanhydrid und Methylamin im Molverhältnis von 1:1,1 einsetzt.

Beispiel 3

Ausführung wie Beispiel 1, jedoch wird ein Katalysator mit 82% NiO, 1,2% CaO, 0,3% MgO, 0,1% $Fe_2O_3$, 8,5% $SiO_2$ und 3% $Al_2O_3$ eingesetzt. Die Ergebnisse sind in der Tabelle aufgeführt. Vergleichbare Ergebnisse werden erhalten, wenn man anstelle von N-Methylsuccinimid N-Ethyl- bzw. N-Propylsuccinimid einsetzt.

Beispiel 4

Ausführung wie Beispiel 1, jedoch wird ein Katalysator mit 79% NiO, 0,8% CaO, 0,7% MgO, 10% $SiO_2$ und 6,5% $Al_2O_3$ eingesetzt. Die erhaltenen Ergebnisse sind in der Tabelle aufgeführt.

Beispiel 5

Ausführung wie Beispiel 1, jedoch wird ein Katalysator mit 81% NiO, 0,7% CaO, 0,5% MgO, 0,5% $Cr_2O_3$, 8% $SiO_2$ und 5% $Al_2O_3$ eingesetzt. Die erhaltenen Ergebnisse sind in der Tabelle aufgeführt.

Beispiel 6

Ausführung wie Beispiel 1, jedoch wird ein Katalysator mit 80% NiO, 0,3% $Fe_2O_3$, 10% $SiO_2$ und 7% $Al_2O_3$ eingesetzt. Die erhaltenen Ergebnisse sind in der Tabelle aufgeführt.

Beispiel 7

Ausführung wie Beispiel 1, jedoch wird ein Katalysator mit 81% NiO, 0,4% $Fe_2O_3$, 0,6% $Cr_2O_3$. 9% $SiO_2$ und 6% $Al_2O_3$ eingesetzt. Die erhaltenen Ergebnisse sind in der Tabelle zusammengestellt.

Beispiel 8

Ausführung wie Beispiel 1, jedoch wird ein Katalysator mit 82 % NiO, 0,7 % $Cr_2O_3$, 9 % $SiO_2$ und 6 % $Al_2O_3$ eingesetzt. Die erhaltenen Ergebnisse sind in der Tabelle zusammengestellt.
Die bei den Katalysatorzusammenstellungen an 100 % fehlenden Prozentpunkte beziehen sich auf organ. Bindemittel (z. B. Graphit).

Vergleichsbeispiel A

Ausführung wie in Beispiel 1, jedoch wird anstelle des erfindungsgemäßen Ni-Katalysators ein Katalysator eingesetzt, der 78 % NiO enthält (Träger: 12 % $Al_2O_3$, 17 % $SiO_2$ und 3 % Graphit). Die Ergebnisse sind in der Tabelle aufgeführt.

Vergleichsbeispiel B

Ausführung wie Beispiel 1, jedoch wird der Druck auf 100 bar gesenkt. Die Ergebnisse sind in der Tabelle aufgeführt.

Zusammenstellung der Versuchsergebnisse

| Beispiel | NMPIN[1] % | gamma-BL[2] % | NMP[3] % | NMSI[4] % | HBSA[5] % | BNMSA[6] % | Rest % | Ausbeute Mol-% |
|---|---|---|---|---|---|---|---|---|
| 1 | 1,6 | 0,8 | 84,8 | 2,8 | 3,0 | 2,9 | 4,1 | 87,9 |
| 2 | 3,3 | 0,3 | 81,4 | 2,0 | 3,0 | 5,1 | 4,9 | 85,2 |
| 3 | 1,2 | 1,1 | 83,1 | 3,1 | 3,2 | 3,4 | 4,9 | 86,9 |
| 4 | 1,3 | 0,9 | 82,5 | 3,2 | 3,0 | 4,2 | 4,9 | 86,5 |
| 5 | 2,1 | 0,6 | 83,6 | 2,9 | 3,1 | 3,2 | 4,5 | 87,0 |
| 6 | 1,4 | 0,5 | 82,1 | 3,5 | 2,9 | 3,8 | 5,8 | 86,4 |
| 7 | 2,2 | 1,0 | 82,4 | 3,0 | 2,8 | 3,5 | 5,1 | 86,0 |
| 8 | 1,8 | 0,8 | 82,7 | 3,2 | 3,0 | 4,1 | 4,4 | 86,3 |
| Vergleichsbeispiel: | | | | | | | | |
| A | 0,7 | 2,1 | 70,0 | 17,1 | 5,2 | 3,4 | 1,5 | 73,3 |
| B | 2,1 | 1,1 | 58,4 | 27,6 | 2,8 | 3,1 | 4,9 | 62,8 |

1 = N-Methylpyrrolidin
2 = gamma-Butyrolacton
3 = N-Methylpyrrolidon
4 = N-Methylsuccinimid
5 = Hydroxi-buttersäure-N-methylamid
6 = Bis-N-Methylsuccinamid

**Patentansprüche**

1. Verfahren zur Herstellung von $C_1$- bis $C_6$-N-Alkylpyrrolidonen aus Bernsteinsäureanhydrid und $C_1$- bis $C_6$-N-Alkylaminen bzw. $C_1$- bis $C_6$-N-Alkylsuccinimid, dadurch gekennzeichnet, daß man Bernsteinsäureanhydrid und $C_1$- bis $C_6$-Alkylamine bzw. $C_1$- bis $C_6$-N-Alkylsuccinimid in Gegenwart eines Ni-Hydrierkatalysators, der 0,1 bis 3% von einem oder mehreren Erdalkalioxiden und/oder Eisenoxid und/oder Chromoxid enthält, bei Temperaturen von 180 bis 250°C und Drücken oberhalb von 100 bar hydriert.

2. Verfahren nach Anspruch 1, dadurch gekennzeichnet, daß man einen Ni-Katalysator einsetzt, der 1,5 bis 2% Erdalkalioxide und/oder Eisenoxid und/oder Chromoxid enthält.

3. Verfahren nach Anspruch 1 und 2, dadurch gekennzeichnet, daß man einen Ni-Katalysator einsetzt, der Erdalkalioxide, Eisenoxid und Chromoxid enthält.

4. Verfahren nach einem der Ansprüche 1 bis 3, dadurch gekennzeichnet, daß man bei Temperaturen von 200 bis 230°C hydriert.

5. Verfahren nach einem der Ansprüche 1 bis 4, dadurch gekennzeichnet, daß man bei Drücken von 250 bis 320 bar arbeitet.

**Claims**

1. A process for the preparation of $C_1$- to $C_6$-N-alkylpyrrolidones from succinic anhydride and $C_1$- to $C_6$-alkylamines, or $C_1$- to $C_6$-N-alkylsuccinimide, characterized in that succinic anhydride and $C_1$- to $C_6$-alkylamines, or $C_1$- to $C_6$-N-alkylsuccinimide, are hydrogenated at temperatures of from 180 to 250°C and pressures of above 100 bar in the presence of a Ni hydrogenation catalyst which contains 0.1 to 3% of one or more alkaline earth metal oxides and/or iron oxide and/or chromium oxide.

2. A process according to claim 1, characterized in that a Ni catalyst is used which contains 1.5 to 2% of alkaline earth metal oxides and/or iron oxide and/or chromium oxide.

3. A process according to either of claims 1 and 2, characterized in that a Ni catalyst is used which contains alkaline earth metal oxides, iron oxide and chromium oxide.

4. A process according to any of claims 1 to 3, characterized in that the hydrogenation is carried out at temperatures of from 200 to 230°C.

5. A process according to any of claims 1 to 4, characterized in that it is operated at pressures of from 250 to 320 bar.

**Revendications**

1. Procédé de préparation de N-alkyl-pyrrolidones en $C_1$ à $C_6$ à partir d'anhydride de l'acide succinique et de N-alkylamines en $C_1$ à $C_6$ ou d'un N-alkyl-succinimide en $C_1$ à $C_6$, caractérisé par le fait qu'à des températures de 180 à 250°C et sous des pressions supérieures à 100 bars, on hydrogène de l'anhydride de l'acide succinique et des alkylamines en $C_1$ à $C_6$ ou un N-alkyl-succinimide en $C_1$ à $C_6$, en présence d'un catalyseur d'hydrogénation au nickel qui renferme de 0,1 à 3% d'un ou plusieurs oxydes alcalino-terreux et/ou d'oxyde de fer et/ou d'oxyde de chrome.

2. Procédé selon la revendication 1, caractérisé par le fait que l'on utilise un catalyseur au nickel qui renferme de 1,5 à 2% d'oxydes alcalino-terreux et/ou d'oxyde de fer et/ou d'oxyde de chrome.

3. Procédé selon les revendications 1 et 2, caractérisé par le fait que l'on utilise un catalyseur au nickel qui renferme des oxydes alcalino-terreux, de l'oxyde de fer et de l'oxyde de chrome.

4. Procédé selon l'une des revendications 1 à 3, caractérisé par le fait qu'on effectue l'hydrogénation à des températures de 200 à 230°C.

5. Procédé selon l'une des revendications 1 à 4, caractérisé par le fait que l'on travaille sous des pressions de 250 à 320 bars.